# EUROPÄISCHE PATENTSCHRIFT

(11) **EP 0 748 787 B1**
(45) Veröffentlichungstag und Bekanntmachung des Hinweises auf die Patenterteilung: **15.12.1999**
(21) Anmeldenummer: 96107513.2
(22) Anmeldetag: 10.05.1996
(51) Int. Cl.: C07C 51/41, C07C 51/295, C07C 53/126

(54) **Verfahren zur kontinuierlichen Herstellung von Monocarbonsäuren**
Process for the continuous preparation of monocarboxylic acids
Procédé pour la préparation continue d'acides monocarboxyliques

(30) Priorität: 16.06.1995 DE 19521900
(43) Veröffentlichungstag der Anmeldung: 18.12.1996
(73) Patentinhaber: RWE-DEA AKTIENGESELLSCHAFT FÜR MINERALOEL UND CHEMIE, 22297 Hamburg (DE)
(72) Erfinder: Herrmann, Albert Thomas, 25541 Brunsbüttel (DE); Meyer, Arnold, 25693 St. Michaelisdonn (DE); Scherf, Erich, 25541 Brunsbüttel (DE); Schröder, Clemens, 23863 Kayhude (DE); Reichenauer, Ansgar, 25709 Marne (DE); Tönsen, Ernst, 25541 Brunsbüttel (DE)
(74) Vertreter: Schupfner, Gerhard D., Dr. Dipl.-Chem.

(56) Entgegenhaltungen:
- DE-A- 4 220 774
- DE-A- 4 302 463
- GB-A- 1 373 275

## Beschreibung

Die Erfindung bezieht sich auf ein Verfahren zur kontinuierlichen Herstellung von linearen und verzweigten gesättigten Monocarbonsäuren mit 6 bis 48 Kohlenstoffatomen. Die linearen bzw. verzweigten primären Alkohole werden in Gegenwart von Alkalihydroxid bei erhöhter Temperatur in einer Alkalischmelze in die Salze der entsprechenden Monocarbonsäuren überführt. In einem zweiten Reaktionsschritt werden aus den Alkalisalzen durch Neutralisation die linearen bzw. verzweigten Monocarbonsäuren freigesetzt.

Das Reaktionsprodukt der obigen Umsetzung, die C6-C48 Monocarbonsäuren, sowie deren Salze, Ester oder Amide sind für vielfältige Anwendungsbereiche geeignet z.B. als Textilzusatzstoffe, Seifen, Tenside, Lösungsmittel, Weichmacher, Hydrauliköle, Schmierstoffe sowie für Epoxid- und Alkydharze.

Die oxidative Herstellung von Salzen der Carbonsäuren in einer Alkalischmelze ist ein seit langem bekannter Prozeß. In der Patentliteratur finden sich zahlreiche Schriften, die sich mit technischen Verfahren zur Verbesserung der Durchführbarkeit dieser Reaktion befassen. Die erforderlichen hohen Temperaturen von 200-370°C in Verbindung mit der stark oxidativen Umgebung durch die Alkalischmelze machen speziell ausgekleidete Reaktoren erforderlich. Bei der Reaktion wird Wasserstoff frei, der explosionssicher abgeführt werden muß. Die starke Gasentwicklung führt zu einem Aufschäumen des Reaktionsproduktes, das damit schlecht handhabbar, insbesondere schlecht rührbar wird. Gleichzeitig verschlechtert sich auch die Raum-Zeit-Ausbeute drastisch.

Die Alkalisalze werden meist in wäßriger Lösung zugesetzt. Die Umsetzung erfolgt ggf. in Gegenwart eines Katalysators wie Zn oder ZnO. Bei den hohen Reaktionstemperaturen wird dem Reaktionsmedium Wasser entzogen, das meist gleichzeitig mit dem Wasserstoff aus dem Reaktor ausgeschleust werden muß. Ohne Lösungsmittel erstarrt das Alkalisalz der Carbonsäure zu einem pastenförmigen Feststoff. Die Rührung und der Stoffaustausch wird gegen Ende der Reaktion zunehmend erschwert, so daß die Reaktion nicht zu sehr hohen Umsätzen geführt werden kann. Nach beendigter Reaktion ist bei hohem Umsatz das Reaktionsgut nicht mehr ohne weiteres, insbesondere nicht mehr kontinuierlich aus dem Reaktor zu entfernen.

Die EP 0 031 694-A1 beschreibt ein Verfahren bei dem zusätzlich ein inertes Verdünnungsmittel z.B. ein Mineralöl zugesetzt wird. Als Produkt wird eine Emulsion des Salzes mit dem Mineralöl erhalten. Nach der Neutralisation, z.B. durch Mineralsäuren liefert die Phasentrennung der wäßrigen Phase von der organischen Phase eine Produktphase, die nun aufwendig gereinigt werden kann, weil sie mit dem inerten Verdünnungsmittel verunreinigt ist.

Auch in der DE 43 02 463-A1 werden in einem Rührreaktor Alkalihydroxid und Guerbet-Alkohole in einer "Batch"-Reaktion zu den entsprechenden Carbonsäuresalzen umgesetzt. Gegen Ende der Reaktion bei hohem Umsatz wird ein inertes Verdünnungsmittel in den Reaktor gesprüht, bevorzugt Wasser. Durch die Wasserzugabe bzw. die Kondensation des Wassers im Dephlegmator wird die Reaktionsmischung gekühlt und die Viskosität erniedrigt. Das Reaktionsgemisch wird so im Rührreaktor handhabbar gehalten. Danach kann das Carbonsäuresalz mit Mineralsäure gespalten werden. Die Reaktion im Rührkessel weist eine unbefriedigend niedrige Raum-Zeit-Ausbeute auf.

Der Erfindung liegt die Aufgabe zugrunde, die im Stand der Technik geschilderten Nachteile - niedrige Raum-Zeit-Ausbeute - schlechte Handhabbarkeit durch hohe Viskosität, Erstarren des Carbonsäuresalzes und Blockieren der Rührung bzw. erschwertes Ausbringen des Reaktionsgutes aus dem Reaktor zu vermeiden und insbesondere ein Verfahren bereitzustellen, das bei hohen Umsätzen kontinuierlich betrieben werden kann , nicht des Zusatzes irgendeines Verdünnungsmittels oder Fließverbesserers bedarf und den entstehenden Wasserstoff in geeigneter Weise abführt, um so ein Aufschäumen des Reaktionsgutes zu vermeiden.

Diese Aufgabe wird erfindungsgemäß gelöst durch Verfahren zur Herstellung von linearen und verzweigten gesättigten Monocarbonsäuren mit 6 bis 48 Kohlenstoffatomen, wobei man (A) lineare bzw. verzweigte primäre Alkohole in Gegenwart von Alkalihydroxid bei erhöhter Temperatur in die Alkalisalze der entsprechenden Monocarbonsäuren überführt und
(B) aus den Alkalisalzen durch Neutralisation die linearen bzw. verzweigten Monocarbonsäuren freisetzt, dadurch gekennzeichnet, daß man (a) die Reaktion zwischen Alkalihydroxid und Alkohol kontinuierlich in einem Mischreaktor durchführt, der das Reaktionsgut neben der Durchmischung vom Eintragsbereich zum Austragsbereich fördert (Reaktionsextruder) und (b) das Reaktionsgut entweder hoch konzentriert einbringt oder während der Reaktion im Mischer durch den Entzug von Wasser und/oder einem anderen Verdünnungsmittel und Wasserstoff entlang der Reaktionsstrecke aufkonzentriert, so daß die Alkalisalze der entsprechenden Monocarbonsäuren pastenförmig als Feststoff ausgetragen werden und sodann die Monocarbonsäure aus ihren Salzen in bekannter Weise durch Neutralisaton freisetzt.

Sodann kann man den pastenförmigen Reaktoraustrag direkt zur Freisetzung der Carbonsäuren in eine Mineralsäure einbringen. Diese Mineralsäure ist bevorzugt verdünnte Schwefelsäure.

Die Alkalihydroxide, bevorzugt NaOH und/oder KOH, werden bevorzugt als 50 bis 86%ige Lösung/Feststoff (Gew.%), im 0 - 40 mol% Überschuß, bevorzugt 20 mol%, gegenüber dem zu oxidierenden Alkohol in den Reaktor im vorderen Bereich des Reaktionsextruders zusammen oder getrennt mit dem Alkohol eingebracht. Dabei können die Edukte vor in den Reaktor auf die Reaktionstemperatur vorgewärmt werden.

Die Reaktion wird bevorzugt bei Temperaturen von maximal 320°C, bevorzugt bei 280-300°C, durchgeführt. Der verdampfende Alkohol und das Wasser aus dem Reaktionsgut kann an einer Stelle oder mehreren Stellen des Reaktionsweges aus dem Reaktionsextruder entfernt werden, wobei der Alkohol kondensiert wird und der Reaktion der Alkohol an einer Stelle oder mehreren Stellen entlang des Reaktionsweges wieder zugeführt werden kann. Ebenso wird der bei der Reaktion entstehende Wasserstoff kontinuierlich an einer oder mehreren Stellen entlang der Reaktionsweges entfernt. Die Oxidation in der Alkalischmelze kann unter Druck durchgeführt werden, wobei der Druck bis zu 30 bar, insbesondere von 2 bis 6 bar, beträgt.

Um die Länge des Reaktionsextruders zu begrenzen und die Verweilzeit zu verkürzen ist es vorteilhaft, in einer Vorreaktion die Alkohole mit einer 50 - 86% wäßrigen Lösung der Alkalihydroxide zu etwa 30 - 70% teilumzusetzen und das unvollständig umgesetzte Reaktionsprodukt in den Mischreaktor einzubringen und dort ggf. weiteres Alkalihydroxid hinzuzufügen. Die Vorreaktion kann bei einem Druck von bis zu 30 bar, besonders bevorzugt bei 2 bis 6 bar, und bei einer Temperatur von bis zu 320°C, besonders bevorzugt bei 280-300°C, durchgeführt werden.

Um ein Verdicken des Reaktionsgutes zu verhindern und zwar bei der Vorreaktion, die vorteilhaft in einem konventionellen Rührreaktor durchgeführt wird, kann das Alkalihydroxid im molaren Unterschuß zugesetzt werden.

Anfänglich vorhandenes Wasser oder der Zusatz eines anderen Verdünnungsmittels, wie z.B. bei der Reaktionsführung mit Vorreaktion vorhanden , ist entweder vor Einbringen des Reaktionsgutes in den Mischreaktor zu entfernen, oder aber die Reaktionsmischung wird entlang des Reaktionsweges, bevorzugt am Anfang der Reaktionsweges im Mischreaktors, vom Verdünnungsmittel befreit, so daß die Reaktionsmischung schon bald eine hochviskose pastenförmige Konsistenz aufweist.

Der Mischreaktor kann als Einwellenknetreaktor ausgebildet sein, eine bevorzugte Ausführungsform des Reaktors ist ein Doppelschneckenextruder. Gegenüber dem Einwellenknetreaktor können die mittleren Verweilzeiten im Doppelschneckenextruder etwa um den Faktor 5 auf unter 2 h verkürzt werden. Ein in Bezug auf den Umsatz optimierte mittlere Verweilzeit im Doppelschneckenextruder ist im allgemeinen gleich oder kleiner als 1 h. Der Mischreaktor ist unter den gegebenen Reaktionstemperaturen alkalibeständig auszuführen. Dies kann z.B. durch eine Ausführung in Nickel gewährleistet werden.

Die Reaktionsweise zeichnet sich dadurch aus, daß alle Verfahrensschritte inklusive der Vorreaktion und der Neutralisation des Carbonsäurealkalisalzes kontinuierlich durchgeführt werden.

### Beispiele:

### Beispiel 1: Darstellung von 2-Butyl-octansäure

In einem kontinuierlich arbeitenden, horizontal angeordneten Einwellen-Knetreaktor wurden, bei einer Temperatur von 320°C und einem Überdruck von 6 bar, 2 kg 2-Butyloctanol / h mit 0,99 kg KOH-Plätzchen (85%ig)/h zur Reaktion gebracht. Die kontinuierliche Zuführung des festen Kaliumhydroxids erfolgte über ein Schleusensystem. Der bei der Reaktion gebildete Wasserstoff wurde über ein Druckhalteventil abgeregelt und mittels einer Gasuhr gemessen. Die mittlere Verweilzeit im Reaktionsapparat lag bei 5 h. Der Austrag der pastenförmigen Alkaliseife erfolgte über eine am Auslaufkopf angeflanschte Zweiwellenaustragsschnecke, die so angeordnet war, daß die ausgetragene Seife in den Rührbehälter fiel. Bei einer Temperatur von 80°C erfolgte unter Rühren die Neutralisation mit 10 %iger Schwefelsäure. Bei einer mittleren Verweilzeit von ≤ 15 Minuten bildete sich die 2-Butyl-octansäure in Form einer organischen Phase, die von der wäßrigen Phase abgetrennt wurde.

Die Reinheit der 2-Butyl-octansäure betrug ≥ 95%.

| Analysendaten: | | |
|---|---|---|
| Säurezahl | [mg KOH/g] | 270 - 290 |
| Esterzahl (max.) | [mg KOH/g] | 3,0 |
| Iodzahl (max.) | [mg l/100 mg] | 2,0 |
| Wasser (max.) | [%] | 0,1 |
| Farbzahl (max.) | (Hazen) | 30 |
| Dichte bei 20°C | [g/ml] | 0,887 |
| Brechungsindex | (bei 20°C) | 1,4394 |
| Schmelzbereich | [°C] | - 14 bis -10 |

### Beispiel 2: Darstellung von 2-Octyl-dodecansäure:

Es wurden 5 kg 2-Octyl-dodecanol/h in einem Rührreaktor bei einer Temperatur von 320°C und einem Überdruck von 2 bar mit 0,94 kg/h einer 50 %igen wäßrigen Kalilauge innerhalb von 0,5h kontinuierlich zur entsprechenden Seife teilumgesetzt. Die erhaltene Seifen- / Alkoholmischung wurde mit 5,44 kg/h in den nachgeschalteten kontinuierlich arbeitenden Zweiwellenextruder gefördert. Hier wurde die restliche 50%ige wässrige Kalilauge mit 1,32 kg/h hinzugegeben. Durch intensives Mischen und Kneten erfolgte die Umsetzung zur Seife mit einer Umsatzrate von ≥ 95%. Unter Normaldruck und bei einer Temperatur von 320°C betrug die Verweilzeit im 40D Extruder ≤ 0,3 h. Der gebildete Wasserstoff und der Wasserdampf aus der Alkalilauge wurde über eine Abgassammelleitung abgeführt, mitgestrippter Alkohol wurde der Reaktion wieder zugeführt . Die Wasserstoffmenge wurde mittels einer Gasuhr zur Reaktionskontrolle gemessen.

Am Extruderaustritt erhielt man eine pastenförmige Seife. Über eine Rohrverbindung fiel die Seife in einen nachgeschalteten Rührbehälter. Bei einer Temperatur von 80°C erfolgte unter Rühren die Neutralisation des Alkalisalzes in 10%iger Schwefelsäure.

Bei einer Verweilzeit von ≤ 15 Minuten erhielt man als organische Phase die 2-Octyldodecansäure, die von der wäßrigen Phase abgetrennt wurde.

Die Reinheit der 2-Octyl-dodecansäure betrug ≥ 97,5%.

| Analysendaten: | | |
|---|---|---|
| Säurezahl | [mg KOH/g] | 170 - 190 |
| Esterzahl (max.) | [mg KOH/g] | 3,0 |
| Iodzahl (max.) | [mg l/100mg] | 2,0 |
| Wasser (max.) | [%] | 0,1 |
| Farbzahl (max.) | (Hazen) | 30 |
| Dichte bei 60°C | [g/ml] | 0,846 |
| Brechungsindex | (bei 60°C) | 1,444 |
| Schmelzbereich | [°C] | 34 - 35 |

### Beispiel 3: Darstellung von 1-Hexansäure

In einen kontinuierlich arbeitenden Zweiwellenextruder wurden 1 kg / h 1-Hexanol gefördert. Über ein Schleusensystem wurden dazu kontinuierlich 0,9 kg/h 85 %ige Kaliumhydroxidplätzchen dosiert. Durch intensives Mischen und Kneten erfolgte die Umsetzung zur Seife mit einer Umsatzrate von ≥ 95%. Bei einer Temperatur von 320°C und einem Druck von 27 bar betrug die Verweilzeit im 40D Extruder ≤ 0,9 h. Der gebildete Wasserstoff und der Wasserdampf aus dem Alkalihydroxid wurden über eine Abgassammelleitung abgeführt, mitgestrippter Alkohol wurde der Reaktion wieder zugeführt. Die Wasserstoffmenge wird mittels einer Gasuhr zur Reaktionskontrolle gemessen. Am Extruderaustritt erhielt man eine feste Seife. Über eine Rohrverbindung fiel die Seife in einen nachgeschalteten Rührbehälter. Bei einer Temperatur von 80°C erfolgte unter Rühren die Neutralisation des Alkalisalzes in 10 %iger Schwefelsäure. Bei einer Verweilzeit von ≤ 5 Minuten erhielt man als organische Phase die Hexansäure, die von der wäßrigen Phase abgetrennt wurde.

Die Reinheit der Hexansäure betrug ≥ 98 %.

| Analysendaten: | | |
|---|---|---|
| Säurezahl | [mg KOH/g] | 470 - 490 |
| Esterzahl (max.) | [mg KOH/g] | 3,0 |
| Iodzahl (max.) | [mg l/100 mg] | 2,0 |
| Wasser (max.) | [%] | 0,1 |
| Farbzahl (max.) | (Hazen] | 10 |
| Dichte bei 20°C | [g/ml] | 0,9286 |
| Brechungsindex | (bei 20°C) | 1,416 |
| Schmelzbereich | [°C] | - 2 bis -1 |

### Beispiel 4: Darstellung von 1-Docosansäure

In einen kontinuierlich arbeitenden Zweiwellenextruder wurden 10 kg/h 1-Docosanol gefördert. Über ein Schleusensystem wurden dazu kontinuierlich 2,8 kg/h 85%ige Kaliumhydroxidplätzchen dosiert. Durch intensives Mischen und Kneten erfolgte die Umsetzung zur Seife mit einer Umsatzrate von ≥ 95 %. Bei einer Temperatur von 320°C betrug die Verweilzeit im 40D Extruder ≤ 0,2 h. Der gebildete Wasserstoff und der Wasserdampf aus dem Alkalihydroxid wurde über eine Abgassammelleitung abgeführt, mitgestrippter Alkohol wurde der Reaktion wieder zugeführt . Die Wasserstoffmenge wurde mittels einer Gasuhr zur Reaktionskontrolle gemessen.

Am Extruderaustritt erhielt man eine pastöse Seife. Über eine Rohrverbindung fiel die Seife in einen nachgeschalteten Rührbehälter. Bei einer Temperatur von 90°C erfolgte unter Rühren die Neutralisation des Alkalisalzes in 10 %iger Schwefelsäure. Bei einer Verweilzeit von ≤ 20 Minuten erhielt man als organische Phase die Docosansäure, die von der wäßrigen Phase abgetrennt wurde.

Die Reinheit der Docosansäure beträgt ≥ 95 %.

| Analysendaten: | | |
|---|---|---|
| Säurezahl | [mg KOH/g] | 155 - 170 |
| Esterzahl (max.) | [mg KOH/g] | 3,0 |
| Iodzahl (max.) | [mg l/100 mg] | 2,0 |
| Wasser (max.) | [%] | 0,1 |
| Farbzahl (max.) | (Hazen) | 100 |
| Dichte bei 90°C | [g/ml] | 0,8225 |
| Brechungsindex | (bei 90°C) | 1,4263 |
| Schmelzbereich | [°C] | 78 - 80 |

### Beispiel 5: Darstellung von 2-Hexadecyl-eicosansäure

In einen kontinuierlich arbeitenden Zweiwellenextruder wurden 10 kg / h 2-Hexadecyleicosanol gefördert. Über ein Schleusensystem wurden dazu kontinuierlich 1,75 kg / h 85 %ige Kaliumhydroxidplätzchen dosiert. Durch intensives Mischen und Kneten erfolgte die Umsetzung zur Seife mit einer Umsatzrate von ≥ 95%. Bei einer Temperatur von 320°C betrug die Verweilzeit im 40D Extruder ≤ 0,2 h. Der gebildete Wasserstoff und der Wasserdampf aus dem Alkalihydroxid wurden über eine Abgassammelleitung abgeführt, mitgestrippter Alkohol wurde der Reaktion wieder zugeführt . Die Wasserstoffmenge wurde mittels einer Gasuhr zur Reaktionskontrolle gemessen.
Am Extruderaustritt erhielt man eine pastöse Seife. Über eine Rohrverbindung fiel die Seife in einen nachgeschalteten Rührbehälter. Bei einer Temperatur von 90 °C erfolgte unter Rühren die Neutralisation des Alkalisalzes in 10 %iger Schwefelsäure. Bei einer Verweilzeit von ≤ 25 Minuten erhielt man als organische Phase die 2-Hexadecyl-eicosansäure, die von der wäßrigen Phase abgetrennt wurde.
Die Reinheit der 2-Hexadecyleicosansäure betrug 90%.

| Analysendaten: | | |
|---|---|---|
| Säurezahl | [mg KOH/g] | 98 - 108 |
| Esterzahl (max.) | [mg KOH/g] | 3,0 |
| Iodzahl (max.) | [mg l/100 mg] | 2,0 |
| Wasser | [%] | 0,1 |
| Farbzahl (max.) | (Hazen) | 200 |
| Dichte bei 80°C | [g/ml] | 0,826 |
| Brechungsindex | (bei 80°C) | 1,4414 |
| Schmelzbereich | [°C] | 70 - 74 |

### Beispiel 6: Darstellung von 2-Butyl-octansäure

In einem kontinuierlich arbeitenden Zweiwellenextruder wurden bei einer Temperatur von 320°C und einem Überdruck von 6 bar, 1 kg 2-Bytyloctanol/h mit 0,84 kg 50%iger Kalilauge/h zur Reaktion gebracht. Der bei der Raktion gebildete Wasserstoff wurde über ein Druckhalteventil abgeregelt und mittels einer Gasuhr gemessen. Die mittlere Verweilzeit im Reaktionsapparat lag bei ca. 1 h. Der Austrag der pastenförmigen Alkaliseife erfolgte über eine angeflanschte Rohrverbindung in einen nachgeschalteten Rührbehälter. Bei einer Temperatur von 80°C erfolgte unter Rühren die Neutralisation mit 10%iger Schwefelsäure. Bei einer Verweilzeit von 15 bis 16 Minuten bildete sich die 2-Butyloctansäure in Form einer organischen Phase, die von der wässrigen Phase abgetrennt wurde.
Die Reinheit der 2-Butyl-octansäure betrug ≥ 95 %.

| Analysendaten: | | |
|---|---|---|
| Säurezahl | [mg KOH/g] | 270 - 290 |
| Esterzahl | [mg KOH/g] | max 3,0 |
| Iodzahl | [mg l/100 mg] | max 2,0 |
| Wasser | [%] | max 0,1 |
| Farbzahl | [Hazen] | max 30 |
| Dichte bei 20°C | [g/ml] | 0,887 |
| Brechungsindex bei 20°C | | 1,4394 |
| Schmelzbereich | [°C] | -14 bis -10 |

## Patentansprüche

1. Verfahren zur Herstellung von linearen und verzweigten gesättigten Monocarbonsäuren mit 6 bis 48 Kohlenstoffatomen, wobei man
(A) lineare bzw. verzweigte primäre Alkohole in Gegenwart von Alkalihydroxid bei erhöhter Temperatur in die Alkalisalze der entsprechenden Monocarbonsäuren überführt und
(B) aus den Alkalisalzen durch Neutralisation die linearen bzw. verzweigten Monocarbonsäuren freisetzt,
**dadurch gekennzeichnet,** daß man
(a) die Reaktion zwischen Alkalihydroxid und Alkohol kontinuierlich in einem Mischreaktor durchführt, der das Reaktionsgut neben der Durchmischung vom Eintragsbereich zum Austragsbereich fördert (Reaktionsextruder) und
(b) das Reaktionsgut entweder hoch konzentriert einbringt oder im Reaktionsgut Alkalihydroxid zusammen mit maximal gleichen Gewichtsteilen Wasser vorliegt und das Reaktionsgut während der Reaktion im Mischer durch den Entzug von Wasser und/oder einem anderen Verdünnungsmittel und Wasserstoff entlang der Reaktionsstrecke aufkonzentriert,
so daß die Alkalisalze der entsprechenden Monocarbonsäuren pastenförmig als Feststoff ausgetragen werden und sodann
die Monocarbonsäure aus ihren Salzen in bekannter Weise durch Neutralisation freisetzt.

2. Verfahren nach Anspruch 1, **dadurch gekennzeichnet,** daß man den pastenförmigen Feststoff-Reaktoraustrag direkt zur Freisetzung der Carbonsäuren in eine Mineralsäure einbringt.

3. Verfahren gemäß einem der vorherigen Ansprüche , **dadurch gekennzeichnet,** daß man in der Verfahrensstufe (A) 50 bis 86%iges wässriges Alkalihydroxid, bevorzugt NaOH und/oder KOH, einsetzt.

4. Verfahren gemäß einem der vorherigen Ansprüche , **dadurch gekennzeichnet,** daß man in der Verfahrensstufe (A) ein 0 - 40%iger molarer Überschuß, bevorzugt ein 20%iger molarer Überschuß, an Alkalihydroxid gegenüber dem Alkohol verwendet.

5. Verfahren gemäß einem der vorherigen Ansprüche , **dadurch gekennzeichnet,** daß man die Reaktion bei Temperaturen von maximal 320°C, besonders bei 280 - 300°C, durchführt.

6. Verfahren gemäß einem der vorherigen Ansprüche , **dadurch gekennzeichnet,** daß man den verdampften Alkohol und das Wasser aus dem Reaktionsgut an einer Stelle oder mehreren Stellen des Reaktionsweges aus dem Mischreaktor entfernt, kondensiert und den Alkohol der Reaktion an einer Stelle oder mehreren Stellen entlang des Reaktionsweges wieder zuführt.

7. Verfahren gemäß einem der vorherigen Ansprüche , **dadurch gekennzeichnet,** daß man den bei der Reaktion entstehenden Wasserstoff kontinuierlich an einer oder mehreren Stellen entlang der Reaktionsweges entfernt.

8. Verfahren gemäß einem der vorherigen Ansprüche , **dadurch gekennzeichnet,** daß man den Verfahrensschritt (A) bei einem Überdruck von bis zu 30 bar, insbesondere von 2 bis 6 bar durchführt.

9. Verfahren gemäß einem der vorherigen Ansprüche , **dadurch gekennzeichnet,** daß man die Reaktion in einem Doppelschneckenextruder durchführt und die mittlere Verweilzeit im Doppelschneckenextruder etwa 1 h oder weniger als 1 h beträgt.

10. Verfahren gemäß einem der vorherigen Ansprüche , **dadurch gekennzeichnet,** daß man in einer Vorreaktion die Alkohole mit einer 50 - 86%igen wäßrigen Lösung der Alkalihydroxide zu etwa 30 - 70 % teilumsetzt und das unvollständig umgesetzte Reaktionsprodukt in den Mischreaktor einbringt und dort ggf. weiteres Alkalihydroxid hinzufügt.

11. Verfahren gemäß Anspruch 10, **dadurch gekennzeichnet,** daß man die Vorreaktion bei einem Überdruck von bis zu 30 bar, insbesondere von 2 bis 6 bar durchführt.

12. Verfahren gemäß einem der Ansprüche 10 oder 11, **dadurch gekennzeichnet,** daß man die Vorreaktion bei einer Temperatur von bis zu 320°C, inbesondere bei 280 bis 300°C, durchführt.

13. Verfahren gemäß einem der Ansprüche 10, 11 oder 12, **dadurch gekennzeichnet,** daß man bei der Vorreaktion das Alkalihydroxid im molaren Unterschuß zusetzt.

14. Verfahren gemäß einem der vorherigen Ansprüche , **dadurch gekennzeichnet,** daß man alle Verfahrensschritte inklusive der Vorreaktion und der Neutralisation des Carbonsäurealkalisalzes kontinuierlich durchführt.

## Claims

1. A process for producing linear and branched saturated monocarboxylic acids having 6 to 48 carbon atoms wherein
(A) linear or branched primary alcohols are converted into the alkali salts of the corresponding monocarboxylic acids in the presence of alkali hydroxide at elevated temperature and
(B) the linear or branched monocarboxylic acids are liberated from the alkali salts by neutralisation,
characterised in that
(a) the reaction of alkali hydroxide and alcohol is continuously carried out in a mixing reactor mixing and conveying the reaction materials from the inlet to the outlet (extrusion reactor) and
(b) the reaction material is either fed in high concentrations, or
alkali hydroxide and maximally equal parts by weight of water are present in the reaction material, and said reaction material is concentrated during reaction in the mixer by removal of water and/or any other diluent and hydrogen along the reaction path such that the alkali salts of the corresponding monocarboxylic acids are discharged paste-like as solid matter and, thereupon,
the monocarboxylic acid is liberated from its salts by known neutralisation methods.

2. A process according to claim 1,
characterised in that the pasty solid discharged from the reactor is directly introduced into a mineral acid in order to liberate the carboxylic acids.

3. A process according to any one of the preceding claims,
characterised in that a 50 to 86 % aqueous alkali hydroxide, preferably NaOH and/or KOH, is used in stage (A) of the process.

4. A process according to any one of the preceding claims,
characterised in that in stage (A) of the process the alkali hydroxide is used in a molar excess of 0 to 40 %, preferably 20 %, based on the alcohol quantity.

5. A process according to any one of the preceding claims,
characterised in that the reaction is carried out at temperatures of max. 320 °C, particularly 280 to 300 °C.

6. A process according to any one of the preceding claims,
characterised in that the vaporised alcohol and the water originating from the reaction material are eliminated from the mixing reactor at one place or several places of the reaction path, are condensed, and the alcohol is returned to the reactor at one place or several places of the reaction path.

7. A process according to any one of the preceding claims,
characterised in that the hydrogen formed during the reaction is continuously removed at one place or several places of the reaction path.

8. A process according to any one of the preceding claims,
characterised in that step (A) of the process is performed at an excess pressure of up to 30 bar, particularly 2 to 6 bar.

9. A process according to any one of the preceding claims,
characterised in that the reaction is carried out in a twin-screw extruder and the average residence time in said twin-screw extruder is approximately 1 hour or less than 1 hour.

10. A process according to any one of the preceding claims,
characterised in that the alcohols are partially reacted at about 30 to 70 % by a preliminary reaction with a 50 to 86 % aqueous solution of the alkali hydroxides and the incompletely reacted product is charged to the mixing reactor to which further quantities of alkali hydroxide may then be added.

11. A process according to claim 10,
characterised in that the preliminary reaction is carried out at an excess pressure of up to 30 bar, particularly 2 to 6 bar.

12. A process according to claim 10 or 11,
characterised in that the preliminary reaction is carried out at a temperature of up to 320 °C, particularly 280 - 300 °C.

13. A process according to any one of claims 10, 11, or 12,
characterised in that for the preliminary reaction the alkali hydroxide is added in hypomolar quantities.

14. A process according to any one of the preceding claims,
characterised in that all the process steps, including preliminary reaction and neutralisation of the carboxylic acid alkali salt, are performed continuously.

## Revendications

1. Procédé de préparation d'acides monocarboxyliques saturés linéaires et ramifiés ayant 6 à 48 atomes, dans lequel :
(A) on convertit des alcools primaires linéaires ou selon le cas ramifiés, en présence d'un hydroxyde alcalin à température élevée, en sels alcalins des acides monocarboxyliques correspondants, et
(B) on libère des sels alcalins, par neutralisation, les acides monocarboxyliques linéaires ou ramifiés,
caractérisé en ce que :
(a) la réaction entre l'hydroxyde alcalin et l'alcool s'effectue en continu dans un réacteur de mélange, qui achemine le matériau réactionnel, tout en effectuant un mélange intime, de la zone d'entrée à la zone de sortie (extrudeuse réactionnelle), et
(b) le matériau réactionnel est soit introduit fortement concentré soit l'hydroxyde alcalin est présent conjointement avec des parties en poids d'eau égales au maximum dans le matériau réactionnel et le matériau réactionnel est concentré au cours de la réaction dans le mélangeur par l'élimination d'eau et/ou d'un autre agent de dilution et d'hydrogène le long du trajet réactionnel, de telle sorte que les sels alcalins des acides monocarboxyliques correspondants soient déchargés sous forme de pâte solide et, qu'ensuite, les acides monocarboxyliques soient libérés de leurs sels de manière connue par neutralisation.

2. Procédé selon la revendication 1, caractérisé en ce que l'on introduit la partie sortant du réacteur sous la forme d'un solide pâteux directement dans un acide minéral pour libérer les acides carboxyliques.

3. Procédé selon l'une quelconque des revendications précédentes, caractérisé en ce qu'on utilise à l'étape (A) du procédé de l'hydroxyde alcalin aqueux à 50% jusqu'à 86%, de préférence du NaOH et/ou du KOH.

4. Procédé selon l'une quelconque des revendications précédentes, caractérisé en ce qu'on utilise à l'étape (A) du procédé un excès molaire de 0-40%, de préférence un excès molaire de 20%, d'hydroxyde alcalin par rapport à l'alcool.

5. Procédé selon l'une quelconque des revendications précédentes, caractérisé en ce qu'on effectue la réaction à des températures de maximum 300°C, en particulier de 280-300°.

6. Procédé selon l'une quelconque des revendications précédentes, caractérisé en ce qu'on élimine, condense, l'alcool vaporisé et l'eau du matériau réactionnel à un ou plusieurs endroits du trajet réactionnel du réacteur de mélange, et on réachemine l'alcool à la réaction à un ou plusieurs endroits le long du trajet réactionnel.

7. Procédé selon l'une quelconque des revendications précédentes, caractérisé en ce qu'on élimine l'hydrogène produit au cours de la réaction en continu à un ou plusieurs endroits le long du trajet réactionnel.

8. Procédé selon l'une quelconque des revendications précédentes, caractérisé en ce qu'on effectue l'étape (A) du procédé à une surpression allant jusqu'à 30 bars, en particulier de 2 à 6 bars.

9. Procédé selon l'une quelconque des revendications précédentes, caractérisé en ce qu'on effectue la réaction dans une extrudeuse à double vis et la durée de séjour moyenne dans l'extrudeuse à double vis est d'environ 1 heure ou de moins de 1 heure.

10. Procédé selon l'une quelconque des revendications précédentes, caractérisé en ce qu'on transforme partiellement, dans une préréaction, les alcools, avec une solution aqueuse à 50-86% des hydroxydes alcalins, à environ 30-70% et on introduit le mélange réactionnel qui n'a pas complètement réagi dans le réacteur de mélange et on y ajoute éventuellement une autre quantité d'hydroxyde alcalin.

11. Procédé selon la revendication 10, caractérisé en ce qu'on effectue la préréaction à une surpression allant jusqu'à 30 bars, en particulier de 2 à 6 bars.

12. Procédé selon l'une quelconque des revendications 10 ou 11, caractérisé en ce que la préréaction est réalisée à une température allant jusqu'à 320°C, en particulier à 280-300°C.

13. Procédé selon l'une quelconque des revendications 10, 11 ou 12, caractérisé en ce qu'on ajoute l'hydroxyde alcalin en sous-excès molaire au cours de la préréaction.

14. Procédé selon l'une quelconque des revendications précédentes, caractérisé en ce qu'on effectue en continu toutes les étapes du procédé, y compris la préréaction et la neutralisation du sel alcalin d'acide carboxylique.
